# EUROPEAN PATENT APPLICATION

(11) **EP 3 912 662 A1**
(43) Date of publication of application: **24.11.2021**
(21) Application number: 20175314.2
(22) Date of filing: 19.05.2020
(51) Int. Cl.: A61M 11/06, A61M 15/00

(54) **NEBULIZER FOR AEROSOLIZING A LIQUID COMPRISING A PIVOTABLE CAP ARRANGED ON THE TOP COVER INCLUDING THE VALVE SYSTEM**

(71) Applicant: Air Liquide Medical Systems S.r.l., 20148 Milano (IT)
(72) Inventor: ALBERICI, Luca, 25073 Bovezzo (IT); RUOCCO, Alessandra, 25073 Bovezzo (IT); SANDONI, Giuseppe, 25073 Bovezzo (IT)
(74) Representative: Air Liquide

(57) **Abstract**

The invention deals with a nebulizer (1) for aerosolizing a liquid, especially a liquid drug, comprising a nebulizer body (2), a top cover (4) fixed to the nebulizer body (2), said top cover (4) comprising a first and a second one-way valve (11, 12) for allowing gas exchanges between the nebulizer body (2) and the atmosphere, a aerosol-generating system for generating an aerosol, a reservoir (5) for storing a liquid to be aerosolized, and a compressed air entry (6) for providing compressed air. The top cover (4) comprises an enlarged opening (7) and a mobile cap (3), said mobile cap (3) being pivotally attached to the top cover (4) and arranged above said enlarged opening (7), and the second one-way valve (12) is arranged on said mobile cap (3).

## Description

The present invention a nebulizer useable in aerosoltherapy comprising a valve system arranged on a top cover attached to the nebulizer body, said valve system comprising an expiratory one-way valve carried by the top cover itself and an inspiratory one-way valve carried by a mobile cap arranged on the top cover above an enlarged opening.

Nebulizing or aerosolizing devices, commonly called nebulizers, are devices useable for delivering an inhalation therapy, i.e. an aerosoltherapy, to a patient in need thereof.

Nebulizers are used in combination with a source of gas, typically compressed air, for nebulizing/aerosolizing a liquid drug solution, for instance a liquid drug or the like, thereby obtaining a mist containing droplets having suitable sizes allowing them to reach the lower airways of the patient that inhales said mist in the course of his/her inhalation therapy or aerosoltherapy.

EP-A-3441097 and EP-A-3441098 propose a breath-enhanced nebulizer comprising a two-valve system including an inhalation and an exhalation valve that is arranged on a detachable cover forming the top, i.e. "roof", of the nebulizer body. The nebulizer is fed with compressed air provided by an external source, such as an air compressor, in fluid communication with the nebulizer. During the inspiration phases of the patient, the inspiratory valve allows extra-air to enter into the internal conduit of the nebulizer for triggering a double Venturi effect, thereby increasing the nebulization rate and consequently the amount of aerosolized drug delivered to the patient, whereas during the exhalation phases, the inspiratory valve closes for lowering the amount of air provided, thereby reducing the nebulization rate and the amount of drug lost in the atmosphere.

Such nebulizers are very efficient for most of the patients.

Nevertheless, it has been noticed that, from time to time, for some particular drugs and/or for some particular patients, for instance some children, it is very important to have a fast therapy, namely to provide large amounts of aerosolized drugs to the patient, even if not "optimized" (i.e. not fine-tuned or less-controlled) in terms of amounts of drug reaching the lungs.

In other words, in some cases, it is less important that some drug-containing mist, i.e. aerosol, can escape and be wasted in the environment, i.e. in the surrounding atmosphere, as in those cases, larger amounts of aerosolized drugs have to be provided for efficiently treating the patients.

A goal of the present invention is to propose an improved nebulizer that is designed for limiting the waste of drug in the surrounding atmosphere when used in a normal or usual situation (i.e. most of the time), but for delivering larger and not "optimized" amounts (i.e. not fine-tuned or less-controlled quantities) of aerosol/mist, i.e. aerosolized drug, when needed/required for allowing an efficient treatment of a patient.

A solution according to the present invention is a nebulizer for aerosolizing a liquid, in particular a liquid drug, i.e. a drug-containing solution, comprising :
- a nebulizer body,
- a top cover fixed, i.e. attached, to the nebulizer body, said cover comprising a first and a second one-way valve for allowing gas exchanges between the nebulizer body and the atmosphere,
- an aerosol-generating system for generating an aerosol,
- a reservoir for storing a liquid to be aerosolized, and
- a compressed air entry for providing compressed air,
characterized in that:
- the top cover comprises an enlarged opening and a mobile cap, said mobile cap being pivotally attached to the top cover and arranged above said enlarged opening, and
- the second one-way valve is arranged on said mobile cap.

In relation to the present invention :
- an "aerosol" is a mist containing droplets of liquid, for instance a liquid drug, and a gas, such as air,
- a "gas" is formed of a unique compound (i.e. a pure gas) or of several compounds (i.e. gas mixture, such as air) that is/are in gaseous form.
- a "liquid drug" or a "drug-containing solution" is a solution that comprises one or several liquid compounds and optionally one or several solid compounds dissolved, suspended or similarly incorporated in said one or several liquid compounds, especially drug compound(s).
- a "cover" means a lid or similar.

Depending on the embodiment, a nebulizer according to the present invention can comprise one or several of the following additional features:
- the top cover is arranged on the top, i.e. roof, of the nebulizer body.
- the mobile cap pivots with respect to the top cover between at least:
   ▪ a closed position (i.e. a first position P1) in which the mobile cap totally closes the enlarged opening, and
   ▪ an open position (i.e. a second position P2) angularly off-set (α) relative to the close position (P1), in which the mobile cap does not totally close the enlarged opening, i.e. the enlarged opening is at least partially open.
- the enlarged opening is fully open when the mobile cap is in the open position.
- the enlarged opening is fully open when the mobile cap is in the open position so that a greater/maximum quantity of air can pass through the enlarged opening and enter into the nebulizer.
- when the enlarged opening is closed by the mobile cap, i.e. closed position, the quantity of air passing through the second one-way valve is limited, i.e. lower.
- the enlarged opening comprises a circular, oval or ellipsoidal section, or the like.
- the enlarged opening comprises a peripheral border arranged in the top cover.
- the mobile cap is sized, i.e. dimensioned, for totally covering the enlarged opening, when it is in the closed position, and ensuring an air tightness between the mobile cap and the peripheral border of the enlarged opening. This obligates air to pass through the second one-way valve, when entering into the nebulizer body.
- the mobile cap pivots between the closed position and the open position, and several angularly off-set intermediary positions comprised between said closed position and open position.
- the mobile cap is pivotable through an angle (α) of less than 180° with respect to the top cover, preferably of between 5° and 120°, typically of between 10° and 90°, typically of about 90°.
- the top cover is detachably fixed to the nebulizer body.
- the mobile cap pivots around a pivot axis (AA) located on the top cover that is perpendicular to a longitudinal axis (XX) of the nebulizer body.
- the mobile cap comprises first attaching means and the top cover further comprises second attaching means, said first attaching means cooperating with said second attaching means for pivotally attaching the mobile cap to the top cover.
- the first attaching means and the second attaching means comprise complementary structures.
- the first attaching means arranged on the mobile cap comprise two lodgings (i.e. holes or the like) and the second attaching means arranged on the top cover comprise two pins (i.e. spikes or the like), or vice versa, said pins being lodged into said lodgings for pivotally attaching the mobile cap to the top cover.
- the mobile cap comprises two lodgings arranged on two parallel walls (i.e. little arms or the like) and the top cover comprises two pins arranged in two parallel slots, said pins being lodged into said lodgings and said parallel walls being lodged into said parallel slots so that said two parallel walls moves into said slots when the two pins pivot into the two lodgings.
- said pins and said lodgings are arranged on the pivot axis (AA).
- each of the two parallel walls further comprises an abutment or the like.
- the mobile cap has an rounded-perimeter, for instance a substantially-oval shape, when seen from above.
- the mobile cap is flat or slightly curved, when seen from the side, preferably curved.
- the mobile cap has an overall saddle-shape.
- the mobile cap has a substantially-oval shape, the first attaching means being arranged at one end of said oval shape mobile cap.
- the second one-way valve arranged on the mobile cap is a one-way inspiration valve configured for allowing additional ambient air entering into the nebulizer body.
- the first one-way valve arranged on the top cover is a one-way expiration valve configured for allowing gas expired by the user being vented to the atmosphere, especially CO₂-containing gas that are exhaled during the expiration phases of the user, i.e. a patient.
- the second one-way valve (i.e. the one-way inspiration valve) is configured for allowing additional ambient air to enter into the nebulization body, during inspiration phases of the user, when the mobile cap is in the closed position (PA), but for preventing any exit/escape of gas through said second one-way valve during the expiration phases of the patient.
- the first one-way expiration valve (i.e. the one-way expiration valve) is configured for allowing gas, especially CO₂-containing gas, expired by the user during the expiration phases of the user, to be vented to the atmosphere, but for preventing any entry of gas through said first one-way valve during the inspiration phases of the user.
- the nebulization body further comprises a nebulization chamber for recovering the aerosol generated by the aerosol-generating system.
- the nebulizer further comprises a respiratory interface fixed to the nebulizer body
- the respiratory interface is in fluid communication with the nebulization chamber for recovering the aerosol flowing out of the nebulization chamber.
- the respiratory interface comprises a mouthpiece, a mouth mask or a facial mask comprising one or several outlets for providing the aerosol to the user, i.e. to the user's airways.
- the compressed air entry is configured for providing compressed air that sucks out liquid to be aerosolized from the reservoir and conveys said liquid towards the aerosol-generating system that is configured for generating an aerosol comprising air and aerosolized liquid.
- the one-way expiration valve (i.e. the first one-way valve) is located between the mobile cap carrying the one-way inspiration valve (i.e. the second one-way valve) and the respiratory interface, when the top cover is fixed to the nebulizer body.
- the reservoir comprises a liquid tank for receiving a liquid to be aerosolized.
- the liquid tank has a cup-like shape.
- the aerosol-generating system comprises a nozzle element comprising a nozzle orifice and a deflector element facing the nozzle orifice.
- the aerosol-generating system comprises a nozzle element for accelerating compressed air provided by the compressed air entry, i.e. air delivered by the source of compressed air, thereby creating a Venturi or suction effect pulling some liquid solution out of the reservoir, namely of the liquid tank.
- the compressed air entry is fluidly connected to the nozzle element by means of a gas conduit or similar.
- the aerosol-generating system comprises deflector element for atomizing the liquid thereby creating a mist containing liquid droplets.
- the nebulizer body further comprises an axially-arranged inner conduit comprising a lower end projecting into the reservoir, in particular into the liquid tank of the reservoir.
- the lower end of the inner conduit terminates close to the bottom of the liquid tank of the reservoir.
- the reservoir further comprises an inner conduit projecting axially and upwardly.
- the axially-arranged inner conduit of the nebulizer body is arranged as a sleeve around the inner conduit of the reservoir.
- the axially-arranged inner conduit of the nebulizer body is spaced from the inner conduit of the reservoir by a spacing of less than 1 mm.
- the nozzle element and the deflector element of the aerosol-generating system are arranged into the lumen of the inner conduit of the nebulizer body.
- the inspiration valve is facing and in fluid communication with an air inlet of the inner conduit axially-arranged in the nebulizer body.
- the nebulization chamber is preferably located above the reservoir, namely above the liquid tank.
- the top cover comprises a grip portion arranged on its outer wall.
- the top cover comprises a textured area forming the grip portion.
- the grip portion is configured for allowing the user to manually-grip said grip portion for easily decoupling/detaching the top cover from the nebulizer body.
- the top cover is press-fitted, snap-fitted or similarly detachably affixable to the nebulizer body.
- the nebulizer body, the reservoir, the mobile cap and/or the top cover are made of polymer, such as PP, PC, ABS, PA, PSU or any other suitable plastic material.
- the respiratory interface comprises an inner passage for the aerosol in fluid communication with the nebulization chamber.
- the respiratory interface comprises a proximal end connected to the nebulizer body and a distal end that is free and comprises the outlet(s), i.e. exit orifice(s).
- the mobile cap further comprises hooking means and the top cover further comprises anchoring means, said hooking means cooperating with said anchoring means for firmly holding the mobile cap in the open position.
- the hooking means arranged on the mobile cap comprise a hook-shape portion and/or the anchoring means of the top cover comprise a outwardly-projecting wall expansion.
- the hook-shape portion and the outwardly-projecting wall expansion have complementary shapes.

A preferred embodiment of a nebulizer according to the present invention is shown in the enclosed Figures, among which:
- Figure 1 is a side view of an embodiment of a nebulizer according to the present invention, with the mobile cap in its close position,
- Figure 2 is similar to Figure 1, except that the mobile cap in its fully open position,
- Figure 3 is a view from above of the mobile cap of the nebulizer of Figures 1 and 2,
- Figures 4 and 5 are side views of the mobile cap of Figure 3,
- Figure 6 is a view from above of the top cover of the nebulizer of Figures 1 and 2,
- Figure 7 is a view from below of the top cover of Figure 6, and
- Figures 8 and 9 are enlarged views of the attachment of the mobile cap to the top cover of the nebulizer of Figures 1 and 2.

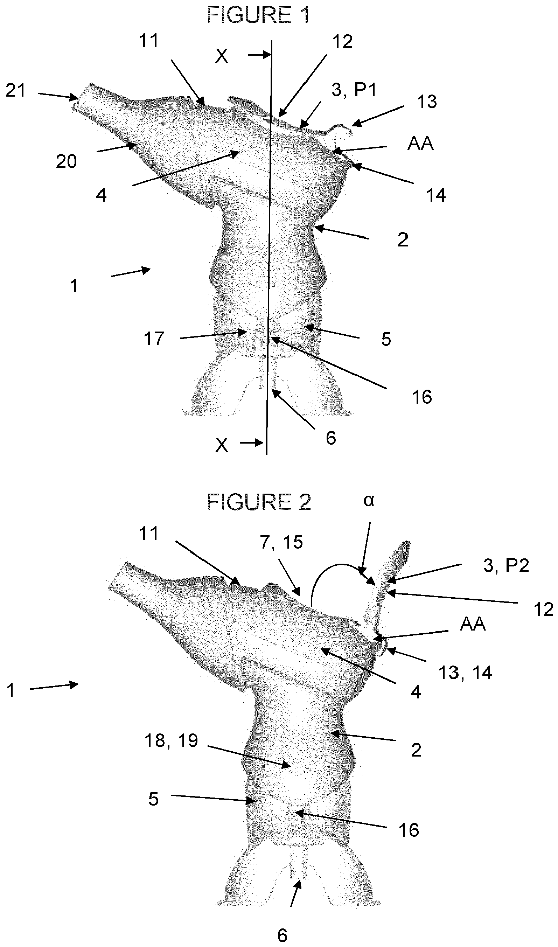

Figures 1 and 2 are side views of an embodiment of a nebulizer 1 according to the present invention, designed for aerosolizing a liquid, such as a drug-containing solution, and thus generating a mist useable in aerosoltherapy.

The nebulizer 1 comprises a reservoir 5 forming the lower part of the nebulizer 1, that is designed for receiving and containing the liquid to be aerosolized, in particular a drug-containing solution, and a nebulizer body 2 arranged above the reservoir 5 comprising a detachable top cover 4 forming the "roof" or top part of the nebulizer body 2.

The nebulizer 1 further comprises an aerosol-generating system (not visible) for generating the aerosol, i.e. the desired mist, and a nebulization chamber for recovering the aerosol, i.e. the mist, generated by the aerosol-generating system, and providing it to a respiratory interface 20, such as a mouthpiece or the like, for delivering the drug-containing mist to a user, i.e. a patient in need thereof, via an outlet 21.

The top cover 4 comprises a valve system 11, 12 and a mobile cap 3.

The reservoir 5 comprises an inner liquid tank 17, also called liquid vessel or liquid compartment, for receiving the liquid to be aerosolized, and further an inner axial conduit 16 arranged so as to project axially and upwardly into the liquid tank 17 of the reservoir 5. Preferably, the tank 17 has a "cup-like" shape. The inner axial conduit 16 is arranged at the center of the liquid tank 17 of the reservoir 5. The role of the inner conduit 16 is to convey a pressurized gas delivered by a gas source (not shown), such as compressed air, fluidly connected to the upstream end of the inner axial conduit 16, namely to a compressed air entry 6 that provides compressed air to the axial conduit 16. The compressed air flow (i.e. pressure > 1 bar abs) travels into the lumen or inner passage of the inner conduit 16 towards the nebulizer body 2 and is delivered by the downstream end (not visible) of the inner axial conduit 16 for creating the mist or aerosol as explained below.

Further, the reservoir 5 is detachably attached to the nebulizer body 2. For instance, they are snap-fitted or threadedly-coupled together, or similarly adapted for repeated attachment/detachment. For instance, they can coupled together by means of a male/female coupling system 18, 19, ensuring a snap-fit connection, which comprises one or several coupling pins 18 arranged on the reservoir 5 that are lodged in one or several complementary coupling lodgings 19 carried by the nebulizer body 2, or vice versa, for instance two coupling lodgings 19 and two coupling pins 18 that diametrally-arranged. The coupling lodgings 19 can be (blind-) bores, i.e. holes, traversing the peripheral wall of the nebulizer body 2. Such a coupling system ensures a good positioning of the reservoir 5 with respect to the nebulizer body 2 as well as an improved fixation of those parts together.

For using the nebulizer 1, the user can grip the nebulizer body 2 as it is shaped and sized for being easily hold in one hand. Grip portions, such as textured areas, can be arranged in the peripheral wall of the nebulizer body 2 for improving the hand grip of the nebulizer 1 by the patient.

Furthermore, the nebulizer body 2 comprises an inner aerosol-generating system for generating the aerosol, i.e. a mist, a nebulization chamber 5 for recovering said aerosol generated by the aerosol-generating system, and an axially-arranged inner conduit comprising an air inlet at its upper end, an air outlet at its lower end and an internal passage arranged in-between for conveying additional ambient air as explained below.

Preferably, at least a part of the nebulization chamber is located between the peripheral wall of the nebulizer body 2 and the axially-arranged inner conduit, and further above the liquid tank 5.

When the reservoir 5 is coupled to the nebulizer body 2, the lower end of the axially-arranged inner conduit of the nebulizer body forms a sleeve around the inner axial conduit of the reservoir 5, that further projects into the liquid tank of the reservoir 3 and terminates close to the bottom of said liquid tank, preferably at a distance of less than about 2 mm. In the aim of allowing a circulation of liquid between them, a little spacing of less than about 1 mm is kept, on the one hand, between the extremity of the axially-arranged inner conduit and the bottom of the liquid tank and, on the second hand, between the inner wall of the axially-arranged inner conduit and the outer wall of the inner axial conduit of the reservoir 5.

Indeed, for generating the aerosol mist, the liquid contained into the tank of the reservoir 5 should be able to circulate in said spacing for reaching the aerosol-generating system that is preferably arranged in the lumen of the inner conduit of the nebulizer body.

The aerosol-generating system can comprise a nozzle element with a small orifice forming a flow restriction, for instance of between about 1 mm to 5 mm, and a deflector element facing said small orifice of the nozzle element, such as a little wall, blade, pin or similar, as known from EP-A-3441097 and EP-A-3441098. Such an aerosol-generating system can generate the aerosol mist thanks to a Venturi effect. Pressurized air provided by the compressed air entry 6 is conveyed and delivered by the inner conduit 16 of the reservoir 5, at a high speed and a constant flowrate. This creates a suction effect that moves the liquid solution out of the tank 14. The liquid solution travels in the spacing, i.e. it is upwardly pulled by the suction effect, and is then delivered by the small orifice of the nozzle element toward the deflector element.

When the liquid enters into contact with the deflector element, a mist of liquid droplets is created by the impact of the liquid on the deflector element.

The smaller droplets create the desired aerosol mist that is recovered into the nebulization chamber, whereas the larger and/or heavier droplets return to the liquid tank.

The aerosol mist can thereafter be inhaled by the patient via a respiratory interface 20 that is fixed to the nebulizer body 2 and is in fluid communication with the nebulization chamber so that the aerosol recovered by the nebulization chamber can be afterwards delivered to the patient. The respiratory interface 20 can be a mouthpiece, or a mouth or facial respiratory mask or similar. The choice of the most suitable interface 20 depends on the type of medication to be taken and on the type of patient, e.g. pediatric patients or adults.

Additional air is generally needed for helping the patient inhaling the aerosol generated by the aerosol-generating system. Said additional airflow is provided by an inspiration one-way valve 12 arranged on a removable top cover 4 as explained below. Additional air enters into the nebulizer body 2 by said inspiration one-way valve 12, travels into the axially-arranged inner conduit and enters into the nebulization chamber 5 thereby boosting the nebulization.

More precisely, the nebulizer body 2 comprises a top cover 4 forming the "top part" or "roof" of the nebulizer body 2 as shown in Figures 1 and 2. The top cover 4 is detachably affixed to the upper portion of the nebulizer body 2, i.e. it removable, for instance for allowing an easy cleaning of the inner parts of the nebulizer body 2.

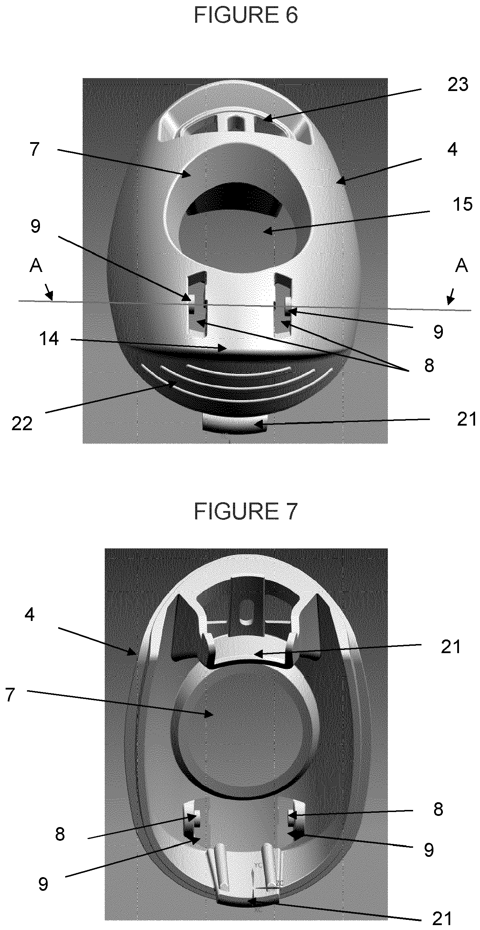

For instance, the removable top cover 4 can be snap-fitted or similarly attached to the nebulizer body 2 by means of connecting structures 21, as visible in Figures 6 and 7, such as pin(s), finger(s), claw(s), clamp(s), hook(s) or the like and corresponding lodging(s), groove(s), slot(s) or the like, that are arranged on the top cover 4 and/or the nebulizer body 2, and cooperate together for allowing a tight and integral affixing of those parts together.

Similarly to the nebulizer body 2, the top cover 4 can comprise a grip portion 22 arranged in its outer wall that allows the user, e.g. the patient, to easily remove the top cover 4 when attached to the nebulizer body 2, for instance for cleaning the nebulization chamber. The grip portion 22 can comprise a textured area, i.e. a not-smooth surface, allowing a good grip to the user, e.g. a textured area comprising "ridges" and "valleys", as shown in Figure 6.

As shown in Figures 1 and 2, the top cover 4 comprises a valve system 11, 12 comprising a pair of one-way valves including a first one-way valve 11, also called one-way expiration valve, allowing gas exiting the nebulizer 1 during the expiration phases of the patient, especially expired gases that contains CO_{2,} and a second one-way valve 12, also called one-way inspiration valve, allowing additional ambient air entering into the nebulizer body 2 and being conveyed by the axially-arranged inner conduit arranged in the nebulizer body 2, during the inspiration phases of the patient, for enhancing the nebulization of the liquid solution and facilitating the inhalation by the patient.

With a nebulizer 1 according to the present invention, the patient does not remove the respiratory interface 20, such as the mouthpiece, out of his/her mouth, before starting to expire CO₂-containing gases as the expired gases can circulate into the respiratory interface 20 before being vented to the atmosphere, through the first one-way valve 11, i.e. an expiration valve.

Preferably, the one-way expiration valve 11, i.e. the first valve, is located between the one-way inspiration valve 12 and the respiratory interface 20. In other words, the expiration valve 11 is located next to the inspiration valve 12, but closer to the mouthpiece 20 than the inspiration valve 12. The one-way expiration valve 11 is mounted into a valve cradle 23 arranged in the top cover 4, as shown in Figure 6.

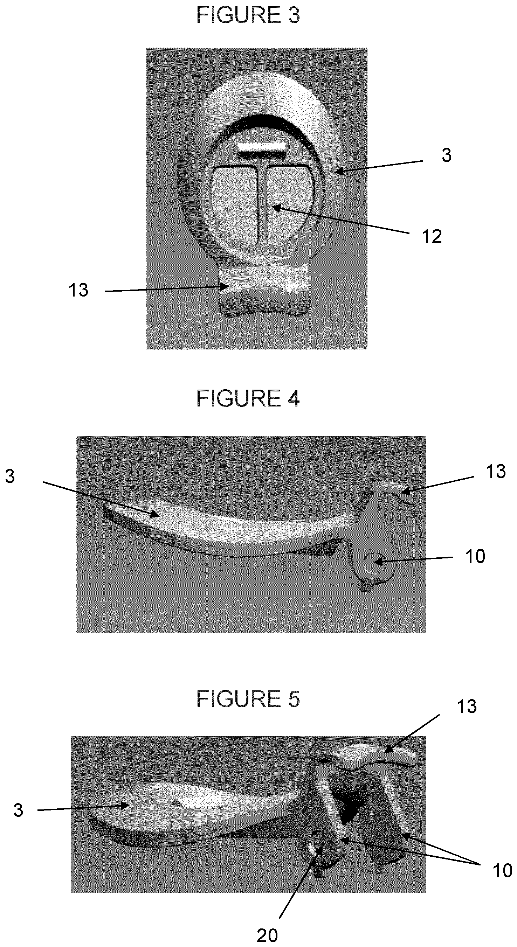

Further, according to the present invention, the inspiration one-way valve 12, i.e. the second one-way valve, is arranged on a mobile cap 3 located on the top cover 4 as shown in Figure 3.

The inspiration one-way valve 12 opens only during inspiration phases of the patient by action of the negative pressure generated by the patient's inspiration, i.e. during inspiration phases, for providing additional air for helping the patient to inhale the aerosol.

Said additional air circulates into the lumen of the axially-arranged inner conduit of the nebulizer body 2. Said additional flow of ambient air is used for boosting the nebulization thanks to a "double" Venturi effect, as already mentioned, thereby significantly increasing the amount of aerosol mist generated by the aerosol-generating system, namely the nebulization rate of the liquid drug, i.e. the solution containing the medication.

Actually, additional ambient air mixes with the aerosol generated by the aerosol-generating system using the compressed air flow provided by the compressed air entry 6 and the liquid sucked out of the reservoir 5.

As, in some particular cases or situations, the amount of additional ambient air provided by the inspiration one-way valve 12, i.e. passing through the inspiration valve 12, is not sufficient, the top cover 4 is further equipped with a mobile cap 3 that may be pivoted, when needed, for increasing the quantity of air that is provided as below explained.

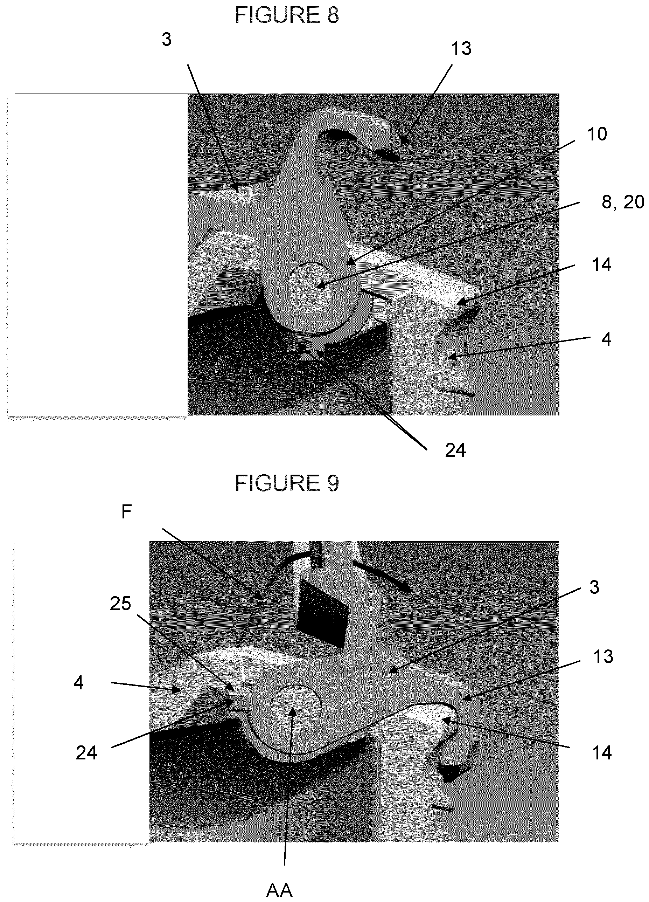

More precisely, the top cover 4 comprises an enlarged opening 7 with the mobile cap 3 arranged on it so as to fully recover said enlarged opening 7, when the mobile cap 3 is in its closed position P1 as shown in Figure 1. However, the enlarged opening 7 can be widely open, when the mobile cap 3 is pivoted by the user, as shown in Figures 2 and 9 (arrow F).

In other words, the mobile cap 3 is pivotally-attached to the top cover 4 that carries the second one-way valve 12, i.e. it can pivot with respect to the top cover 4 between several positions that are angularly off-set. The mobile cap 3 pivots around a pivot axis AA located on the top cover 4 that is perpendicular to the longitudinal axis XX of the nebulizer 1, as shown in Figure 1.

Those positions comprises at least a closed position P1 in which the mobile cap 3 totally closes the enlarged opening 7 (cf. Fig. 1), and an (fully) open position P2 angularly off-set (cf. angle α in Fig. 2) relative to the closed position P1, in which the mobile cap 3 does not close the enlarged opening 7, i.e. the enlarged opening 7 is preferably fully open with the mobile cap 3 maintained in said fully-open position (cf. Fig. 2) by specific fixing means 13, 14, as explained below. Of course, several intermediary angular positions exist between the close position P1 and the open position P2 all along the pivot angle (α).

The pivot angle (α) is generally of less than 180° with respect to the top cover 4), preferably of between 5° and 120°, typically of between 10° and 90°, for instance of about 90° in the embodiment shown in Fig. 2.

For ensuring a pivoting and an efficient attaching of the mobile cap 3, it is provided first attaching means 10, 20 on the mobile cap 3 and further second attaching means 8, 9 on the top cover that cooperate together for pivotally attaching the mobile cap 3 to the top cover 4.

In the embodiment shown in Figures 3-8, the first attaching means 10, 20 arranged on the mobile cap 3 comprise two lodgings 20 and the second attaching means 8, 9 arranged on the top cover 4 comprise two pins 9. The pins 9 are lodged into the lodgings 20 for ensuring both a pivoting and an attachment of the mobile cap 3 to the top cover 4. The pins 9 and the lodgings 20 are located on the pivot axis AA.

The two lodgings 20 are carried by two parallel walls 10 or arms arranged face-to-face on the mobile cap 3 as shown in Figures 4 and 5, whereas the two pins 9 are arranged in two parallel slots 8 carried by the top cover 4 as shown in Figure 6. When the pins 9 are lodged into the lodgings 20, the parallel walls 10 are lodged into the two parallel slots 8 so that they can pivot in said slots 8.

Further, each of the two parallel walls 10 comprises an abutment 24 that cooperates with an inner part 25 of the top cover 4 for limiting the angular course of the mobile cap 3 while it pivots, i.e. each abutment 24 abuts against a corresponding inner part 25 of the top cover 4.

During the inspiration phases of the user, when the mobile cap 3 is in its closed position P1, as shown in Fig. 1, ambient air is forced to pass through the second one-way valve 12 for entering into the nebulizer body 2, whereas when the mobile cap 3 is in the angularly off-set open position P2, as shown in Fig. 2, ambient air enters into the nebulizer body 2 directly via the enlarged opening 7 as said enlarged opening 7 is widely open, i.e. no more closed by the mobile cap 3, which leads to an increase amount of air available for generating the aerosol thereby accelerating the therapy as a greater quantity of air can pass through the enlarged opening 7.

For firmly holding the mobile cap 3 in the open position P2, as shown in Fig. 2, a hooking means 13 on the mobile cap 3 cooperates with anchoring means 14 carried by the top cover 4. In the embodiment shown in Figures 8 and 9, the hooking means 13 arranged on the cover 3 comprise a hook-shape portion, whereas the anchoring means 14 of the nebulizer body 2 comprise an outwardly-projecting wall expansion, which have complementary shapes. Such structures and shapes allow the hook-shape portion of the mobile cap 3 to dock with the outwardly-projecting wall expansion of the mobile cover 4 so that the mobile cap 3 and the mobile cover 4 are integral with one another, with the mobile cap 3 in its open position P2 and the enlarged opening 17 widely open.

As illustrated in Figures 3-5, the mobile cap 3 has preferably a rounded-perimeter, for instance a substantially-oval shape, when seen from above (Fig. 3). It can further be slightly curved, when seen from the side (Fig. 4), namely having an overall saddle-shape (Fig. 4 & 5). Preferably, the first attaching means 10, 20 are arranged at one end of said oval shape mobile cap 3.

All the components of the nebulizer 1, i.e. top cover 4, mobile cap 3, nebulizer body 2, reservoir 5, etc., are preferably made of plastic material(s), such as PP, PC, ABS, PA, PSU or similar materials, while the masks are made of a soft elastomeric material, such as silicone or the like.

Thanks to the nebulizer 1 according to the present invention, the patient can execute his/her aerosoltherapy by both inhaling and exhaling gases through the respiratory interface 20 and the removable cover 4, the amount of aerosolized drug wasted in the environment is considerably reduced, compared with prior art nebulizers, as the exit of expired gases is controlled by the one-way expiration valve 11, i.e. there is no permanent opening for exhaled air.

Further, thanks to the mobile cap 3 carrying the inspiration valve 12, the amount of additional air can be adjusted depending on the position (i.e. open or close) of the mobile cap 3.

The nebulizer 1 of the present invention is usable in combination with a source of gas, such as compressed air, for nebulizing a drug-containing solution and thereby obtaining a mist containing droplets of medication having suitable sizes so as to be able to reach the lower airways of the patient that has to inhale said mist in the frame of his/her aerosoltherapy.

## Claims

1. Nebulizer (1) for aerosolizing a liquid, especially a liquid drug, comprising :
- a nebulizer body (2),
- a top cover (4) fixed to the nebulizer body (2), said top cover (4) comprising a first and a second one-way valve (11, 12) for allowing gas exchanges between the nebulizer body (2) and the atmosphere,
- a aerosol-generating system for generating an aerosol,
- a reservoir (5) for storing a liquid to be aerosolized, and
- a compressed air entry (6) for providing compressed air,
**characterized in that**:
- the top cover (4) comprises an enlarged opening (7) and a mobile cap (3), said mobile cap (3) being pivotally attached to the top cover (4) and arranged above said enlarged opening (7), and
- the second one-way valve (12) is arranged on said mobile cap (3).

2. Nebulizer according to Claim 1, **characterized in that** the mobile cap (3) pivots with respect to the top cover (4) between at least:
- a closed position (P1) in which the mobile cap (3) totally closes the enlarged opening (7), and
- an open position (P2) angularly off-set (α) relative to the closed position (P1), in which the mobile cap (3) does not totally close the enlarged opening (7), i.e. the enlarged opening (7) is at least partially open, preferably fully open.

3. Nebulizer according to any one of the preceding claims, **characterized in that** the mobile cap (3) is pivotable through an angle (α) of less than 180° with respect to the top cover (4), preferably of between 5° and 120°, typically of between 10° and 90°.

4. Nebulizer according to any one of the preceding Claims, **characterized in that** the mobile cap (3) comprises first attaching means (10, 20) and the top cover (4) further comprises second attaching means (8, 9), said first attaching means (10, 20) cooperating with said second attaching means (8, 9) for pivotally attaching the mobile cap (3) to the top cover (4).

5. Nebulizer according to Claim 4, **characterized in that** the first attaching means (10, 20) arranged on the mobile cap (3) comprise two lodgings (20) and the second attaching means (8, 9) arranged on the top cover (4) comprise two pins (9), or vice versa, said pins (9) being lodged into said lodgings (20) for pivotally attaching the mobile cap (3) to the top cover (4).

6. Nebulizer according to Claim 5, **characterized in that** the mobile cap (3) comprises two lodgings (20) arranged on two parallel walls (10) and the top cover (4) comprises two pins (9) arranged in two parallel slots (8), said pins (9) being lodged into said lodgings (20) and said parallel walls (10) being lodged into said parallel slots (8).

7. Nebulizer according to any one of the preceding Claims, **characterized in that** the mobile cap (3) further comprises hooking means (13) and the top cover (4) further comprises anchoring means (14), said hooking means (13) cooperating with said anchoring means (14) for firmly holding the mobile cap (3) in the open position (P2).

8. Nebulizer according to Claim 7, **characterized in that** the hooking means (13) arranged on the mobile cap (3) comprise a hook-shape portion and/or the anchoring means (14) of the top cover (4) comprise an outwardly-projecting wall expansion.

9. Nebulizer according to Claim 8, **characterized in that** the hook-shape portion and the outwardly-projecting wall expansion have complementary shapes.

10. Nebulizer according to any one of the preceding Claims, **characterized in that** the aerosol-generating system comprises a nozzle element comprising a nozzle orifice and a deflector element facing the nozzle orifice.

11. Nebulizer according to any one of the preceding Claims, **characterized in that** the nebulizer body (2) further comprises a nebulization chamber for collecting the aerosol generated by the aerosol-generating system.

12. Nebulizer according to any one of the preceding Claims, **characterized in that** it further comprises a respiratory interface (20) in fluid communication with the nebulization chamber.

13. Nebulizer according to any one of the preceding Claims, **characterized in that** the inspiration valve (12) is facing and in fluid communication with an air inlet of an inner conduit axially-arranged in the nebulizer body (2).

14. Nebulizer according to any one of the Claims 1 to 9, **characterized in that** the mobile cap (3) has a rounded-perimeter, for instance a substantially-oval shape, and/or is curved, preferably the mobile cap (3) has an overall saddle-shape.

15. Nebulizer according to Claim 1 to 9 and 14, **characterized in that** the mobile cap pivots (3) around a pivot axis (AA) located on the top cover (4) that is perpendicular to a longitudinal axis (XX) of the nebulizer body (2).
